Europäisches Patentamt

European Patent Office

Office européen des brevets

(19)

(11) Publication number: **0 387 380**
**A1**

(12) **EUROPEAN PATENT APPLICATION**

(21) Application number: **89104762.3**

(22) Date of filing: **17.03.89**

(51) Int. Cl.5: **C12N 15/00, C12N 9/72**

(43) Date of publication of application:
**19.09.90 Bulletin 90/38**

(84) Designated Contracting States:
**AT BE CH DE ES FR GB GR IT LI LU NL SE**

(71) Applicant: **BEHRINGWERKE Aktiengesellschaft**
**Postfach 1140**
**D-3550 Marburg 1(DE)**

(72) Inventor: **Paques, Eric Paul, Dr.**
**Schmiedeacker 18**
**D-3550 Marburg(DE)**

(74) Representative: **Klein, Otto, Dr. et al**
**Hoechst AG Zentrale Patentabteilung**
**Postfach 80 03 20**
**D-6230 Frankfurt am Main 80(DE)**

(54) Mutants of urinary plasminogen activator, their production and use.

(57) Mutants of urinary plasminogen activator are described having alone or in combination a change of the glycosylation pattern, truncation of the N-terminus, and modification of the cleavage site. These polypeptides are produced by expression of mutated genes created by site directed mutagenesis.

EP 0 387 380 A1

## Mutants of Urinary Plasminogen Activator, Their Production and Use

Summary of the invention

Polypeptides having plasminogen activator activity are provided by introducing deletions or substitutions, individually or in combination in the urinary plasminogen activator (u-PA) molecule. This involves change of the glycosylation pattern, truncation of the N-terminus, and modification of the cleavage site. These polypeptides are produced by expression of mutated genes created by site-directed mutagenesis. Both the single chain form and the activated two chain form of the polypeptides above are useful in the prevention of clot formation and in the dissolution of thrombin.

Field of the invention

Urinary plasminogen activator (u-PA) is a serine protease involved with the dissolution of blood clots. The molecule has a number of distinct regions which display amino acid sequence homology with other naturally occurring polypeptides. Beginning at the N-terminus is a region homologous to various growth factors. The growth factor region is followed by a kringle region. The active site is analogous to other serine proteases.

There are at least two different properties associated with urinary plasminogen activator and its ability to lyse blood clots in vivo. The first is the specific activity of the protease function in cleaving plasminogen to produce plasmin which in turn degrades fibrin. The second property is the sensitivity to inhibition by plasminogen activator inhibitor. Contrary to tissue plasminogen activator (t-PA), u-PA has no affinity to fibrin and its plasminogenolytic activity is very little fibrin dependent by comparison with t-PA. Each of these properties plays a role in the rapidity and the specificity with which plasminogen activator will cleave plasminogen to plasmin in the presence of blood clots. It would therefore be of substantial interest to be able to produce polypeptides having urinary plasminogen activator activity which will provide for improved properties in one or more of these categories.

Brief description of the relevant literature

Husain et al. (PNAS (1981) 78: 4265-4269), report the existence of a proenzym form of urokinase. Steffens et al.,(Hoppe-Seyler's Z. Physiol.Chem. (1982) 363: 1043-1058 and 1155-1165), report the amino acid sequence of urokinase. Ratzkin et al., (PNAS (1981) 78: 3313-3317), report the expression of urokinase in E.coli. Pannell and Gurewich, (Blood (1987) 1: 22-26), report that clipping at the cleavage site of prourokinase is necessary for protease acitivty. Gurewich and Pannell, (Blood, (1987) 3: 769-722), report that thrombin inactivates prourokinase. Stump et al., (JBC (1986) 261: 17120-17126) report the existence of a low molecular form of prourokinase. Lijnen et al., (JBC (1988) 263: 5594-5598) report the effect of removal of the polypeptide $Ser_1$ to $Glu_{143}$ on the prourokinase thrombolytic properties.

Description of the specific embodiments

Novel polypeptides having enhanced properties as plasminogen activators are provided including DNA coding for such polypeptides, expression cassettes containing such sequences for expression in an appropriate host, hosts capable of expressing the novel polypeptides and methods for producing the novel polypeptides. The novel polypeptides involve (1) changes at the glycosylation site (2) at least one change at the cleavage site in the region from 155 to 162, particularly from 159 to 162, (3) truncation at the N-terminus of the molecule, and/or (4) substitution of $His_{56}$ and/or $Ala_{77}$ against Ser or Thr. Where the modification is at the glycosylation site, the glycosylation site at 302-304 will be modified, either at residue 302 or at residue 304. (The numbering of the amino acids which is employed is based on that reported in EP 0 092 182). By introducing each of these changes alone or in combination into polypeptides having plasminogen activator activity and derived from human plasminogen activator improved physiological properties are achieved. The novel polypeptides exhibit one or a combination of the following properties: enhanced proteolytic and specific plasminogenolytic activity, reduced sensitivity to plasminogen activator inhibition, increased resistance towards inactivation by thrombin.

2

EP 0 387 380 A1

The first change of interest is the modification of the glycosylation site at amino acids 302-304, where either the asparagine is changed, conservatively or non-conservatively, or the threonine is changed non-conservatively, particularly by Gly, Ala or Val. Mutations of interest include substituting asparagine with other amino acids, preferably glutamine, valine, leucine, isoleucine, alanine or glycine, more preferably glutamine or substituting serine or threonine with other amino acids, preferably alanine, valine or methionine, that is, aliphatic amino acids of from 3 to 5 carbon atoms lacking mercapto or neutral hydroxyl substituents. Substituting asparagine with glutamine at the glycosylation site at 302 is specially preferred.

Conservative changes are indicated by amino acids included within a semi-colon. G, A; V, I, L; D, E; K, R; N, Q; S, T; and F, W, Y; where an amino acid in one grouping is substituted by an amino acid in another grouping or an amino acid which is not indicated above, such change will be considered non-conservative. Likewise various lesions may be introduced at the glycosylation sites, such as deletions, substitutions, insertions, or the like, to change the glycosylation site triad so as to destroy the glycosylation site.

The second area of interest is modification of the cleavage site which occurs at amino acids 155 to 162. Of particular interest are non-conservative changes, such as substituting $Ile_{160}$ with Arg or Lys. Other substitutions include replacing isoleucine at 159. It was found that a specific modification at the cleavage site will reduce sensitivity to plasminogen activator inhibition, so that in vivo, the polypeptide will have an increased activity as compared to the wild-type plasminogen activator in the presence of a naturally inhibiting amount of plasminogen activator inhibitor.

The third change of interest is deletion of at least 1 amino acid and up to 157 amino acids, in the region in between $Ser_1$ and $Lys_{158}$, usually of the polypeptides in between $Cys_{11}$ and $Lys_{135}$, $Glu_{43}$ and $Lys_{135}$ or $Cys_{50}$ to $Lys_{135}$.

The fourth area of interest is modification of the amino acids 56 and/or 77. Of particular interest are changes, such that new glycosylation sites are generated. This objective is reached by change of the amino acids 56 and/or 77 against Ser of Thr.

The novel polypeptides of the invention are produced by combining two, three or more of the changes described above. For example, in addition to substituting asparagine with glutamine at the glycosylation site, at 302, an example of the first type of change, the cleavage site at 160 may be modified by substituting isoleucine with arginine or lysine and/or the sequence N-terminal amino acid sequence may be truncated.

In some instances, rather than truncating the u-PA its chain may be extended or terminal amino acids added, generally from about 1 to 12 amino acids at one or both of the N- and C-termini. Added amino acids may serve as linkers, provide a particular immune response, modify the physical properties of the molecule, or the like. The DNA sequences encoding for the subject polypeptides may be prepared in a variety of ways, using chromosomal DNA including introns, cDNA, synthetic DNA, or combinations thereof. The desired mutations may be achieved by cloning the gene in an appropriate bacterial host using a single-stranded bacteriophage vector or double-stranded plasmid vector and then using a mismatched sequence substantially complementary to the wild-type sequence but including the desired mutations, such as deletions or insertions. Alternatively, where convenient restriction sites exist one can excise a particular sequence and introduce a synthetic sequence containing the mutations. Other techniques which may be employed are partial exonucleolytic degestion of a double-stranded template or in vitro heteroduplex formation to expose partially single-stran ded regions for oligonucleotide-directed mutagenesis. The preferred method here is oligonucleotid directed mutagenesis. (Zoller and Smith, Methods Enzymol. (1983) 100: 468-500). Appropriate oligonucleotide sequences containing the mismatches, deletions or insertions are prepared generally having about 20 to 100 nucleotides (nt), more usually 20 to 60nt. The sequences can be readily prepared by synthesis and be cloned in an appropriate cloning vector. The oligonucleotides are annealed to a purified template of the u-PA gene or u-PA analog for site directed mutagenesis. Mutations at different sited may be introduced in a single step or successive steps, after each mutation establishing the existence of the mutation and its effect on the activity of the u-PA analog.

Conveniently, the vector used for mutation is a single-stranded circular DNA virus, e.g., M13, particularly where modified as e.g. M13mp9. The mutagenesis primer is combined with the template and vector under annealing conditions in the presence of an appropriate polymerase lacking correction capability, such as the Klenow fragment or T4 DNA polymerase in the presence of the necessary nucleotides and T4 ligase, whereby the cyclic, gene carrying vector is replicative to provide for the double-stranded replication form. The double-stranded DNA may then be introduced into an appropriate cell. By providing for a lytic vector, plaques are obtained which may be screened with a primer for the presence of the mutagenized u-PA or u-PA analog. Usually, at least two repetitive screenings will be required to enhance the probability of having the correct sequence for the mutagenized product. The putative positive clones are then purified, e.g., plaque purified, electrophoretically purified, or the like, and subsequently sequenced.

3

Once the correctly mutagenized gene is obtained, it is isolated and inserted into an appropriate expression vector for expression in an appropriate host. A wide variety of expression vectors exist for use in mammalian, insect and avian hosts. Of particular interest are mammalian expression vectors, which employ viral replicons, such as simina virus, bovine papilloma virus, adenovirus, or the like. The transcriptional and translational initiation and termination regulatory regions are preferably the regions naturally associated with the u-PA gene or regions derived from viral structural genes or regions derived from host genes. A large number of promoters are available, such as the SV40 early and late promoters, adenovirus major late promoter, β-actin promoter, human CMV Immediate Early (IE) promoter, etc. Desirably, the promoters will be used with enhancers to provide for improved expression. In many instances it will be desirable to amplify the u-PA expression construct.

For amplification or for other reasons, it may be desirable to have the expression construct integrated into the genome. Towards this end the construct will either be joined to an unstable replication systeme, e.g., yeast arsl, or lack a replication system. For integration, see, for example, Axel and Wigler, U.S. Patent No. 4,399,216. Usually a marker expression construct will be in tandem with the subject expression construct, which marker may be the same or different from the amplifying gene. This can be achieved by having the u-PA in tandem with an expression construct, functional in the same host as the u-PA expression construct, and encoding such amplifiable genes as DHFR (dihydrofolate reductase), TK (thymidine kinase), MT-I and -II (metallothionein, e.g., human), ODC (ornithine decarboxylase) or GS (glutamine synthetase). By stressing the host in accordance with the amplifiable gene, e.g. methionine sulphoximine with GS, the subject expression constructs may be amplified. Depending upon the particular hosts and signal sequences used, the u-PA analog which is expressed is retained in the host cell in the cytoplasm or is secreted. Where retained in the cytoplasm, the u-PA analog is isolated by lysis of the host cell using standard methods and purified by extraction and purification, preferably by employing electrophoresis, chromatography, or the like. Where the u-PA is secreted, the secreted u-PA is isolated from the supernatant in accordance with conventional techniques including affinity chromatography.

The properties of the mutant polypeptides disclosed in this invention are superior to the naturally occurring plasminogen activator u-PA. The subject compounds will be superior to wild-type u-PA in at least one of the following aspects.

a) increased specific activity, b) reduced susceptibility to inhibition by human plasminogen (c) reduced susceptibility to inactivation by thrombin, (d) longer half-live in vivo. (See Examples for test procedures).

The subject polypeptides may be used in a variety of ways in the prophylactic or therapeutic treatment for various vascular conditions or diseases specifically to protect a mammalian, particularly a human host from thrombus formation. They may therefore be administered during operations where the host may be susceptible to blood clotting, or for treatment of thrombotic patients, to dissolve blood clots which may have formed in deep vein thrombosis, pulmonary embolism, cranial and coronary thrombosis.

The subject compounds may be administered enterally or parenterally, especially by injection or infusion. These compounds may be administered in an effective amount in a physiologically acceptable carrier, such as water, saline, or appropriate buffer systems in the presence of protein stabilizing substances selected from the group of gelatin, gelatin derivatives, protective proteins such as albumin, sugar, sugar alcohol, or amino acids solely or in combination with other therapeutic drugs, especially for vascular diseases. It is evident from the above results that substantial advantages can be achieved by making changes in the wild-type u-PA amino acid sequence. Thus, not only the resistance to thrombin inactivation can be increased, but at the same time sensitivity to plasminogen activator inhibitor can be decreased, so that overall a very substantial increase in effective activity can be achieved in vivo. Thus, one can administer lower amounts of these polypeptides with plasminogen activator activity, so as to minimize the level in the blood stream of the protein (or enzyme) and substantially diminish the undesirable side effects of u-PA, while providing for increased activity against clots.

Although the foregoing invention is described in some detail by way of illustration and examples for purposes of clarity of understanding, it will be obvious that certain changes and modifications may be practised within the scope of the appended claims defining also the instant invention.

Examples

Example 1: General mutagenesis procedures

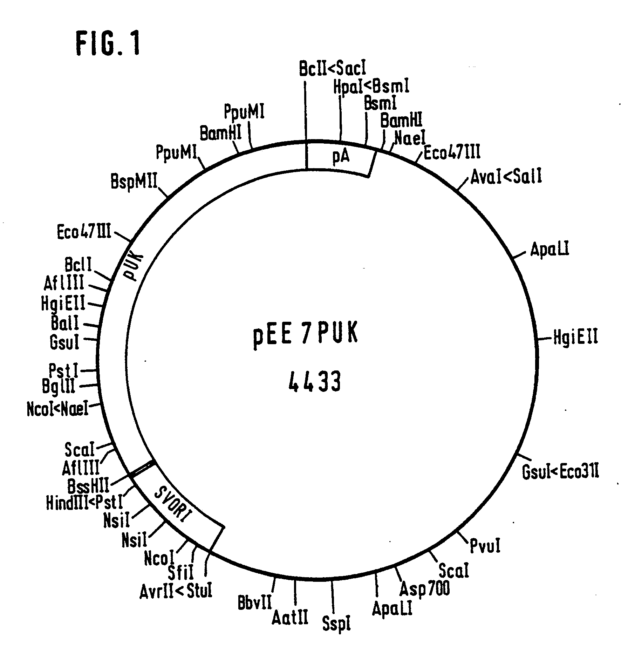

Sequencing primers were designed for sequencing by the dideoxynucleotide chain termination method in bacteriophage M13 (F. Sanger, S. Nicklen and A.R. Coulson, Proc. Natl. Acad. Sci. USA (1977) 74: 5463). The sequencing primers were designed to be complementary to M13mp8 recombinant templates, of 19 or 20 bases in length and to anneal at a position at least 50 bases away from the site of the mutation. The vector used for all mutagenesis procedures was a plasmid capable of expressing u-PA in mammalian cells, pEE7 pUK shown in Figure 1.

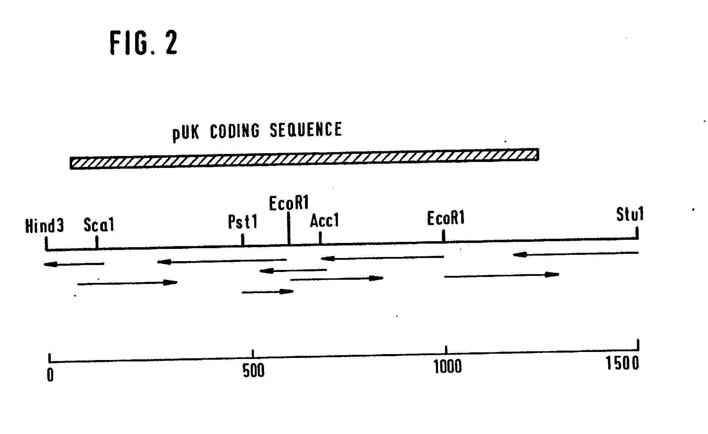

pEE7 pUK was made as follows. A 1.5 kb HindIII-StuI fragment from a recombinant lambdagt10 phage clone, H1.2, containing a partial u-PA cDNA (Figure 2) was subcloned into HindIII-SmaI digested pSP64 (Melton et al. Nucleic Acids. Res. 12: 7035 (1984) to form pSP64 H1.2. This plasmid was digested with HindIII and ScaI and the larger of the two resulting fragments was ligated to chemically synthesised oligonucleotide to form pSP64.pUK. This plasmid then contains a complete pre-pro-urokinase open-reading frame and a short 5'-untranslated sequence which provides a consensus translation-initiation signal for mammalian cells and HindIII and Pst-1 restriction endonuclease recognition sites at the 5'-end for convenient insertion into expression vectors.

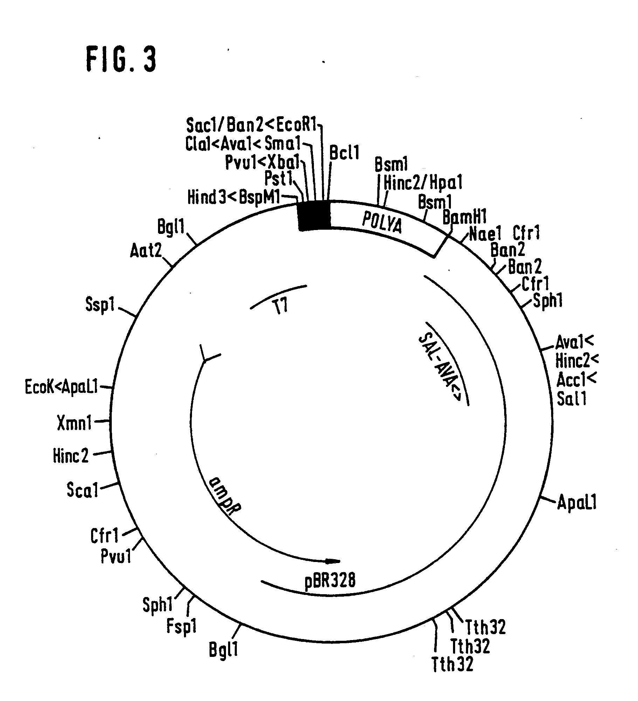

The sequence of the pre-pro-urokinase open reading frame is shown in EP-A2- 0 092 182. The 1.5 kb HindIII-SacI fragment of pSP64.PUK (which includes the entire sequence shown in table 4A ibidem) was inserted between the HindIII and Sac1 sites of pEE7, to form pEE7.pUK. PEE7 was derived by insertion of the 342 bp PvuII-HindIII fragment of SV40, (SV40 nucleotides 5171-270) containing the origin of replication, into the HindIII site of pEE6 such that the SV40 late promoter directs transcription towards the polylinker of pEE6. pEE6 is a bacterial vector from which sequences inhibitory to replication in mammalian cells have been removed (see Fig. 3). It contains the XmnI to BclI portion of pCT54 (Emtage et al. (1983) Proc. Natl. Acad. Sci. USA 80, 3671-3675) with a pSP64 (Melton et al. (1984) Nucleic Acids. Res. 12, 7035) polylinker inserted in between the HindIII and EcoRI sites. The BamHI and SalI sites have been removed from the polylinker by digestion, filling in with Klenow polymerase and religation. The BclI to BamHI fragment is a 237 bp SV40 early polyadenylation signal (SV40 2770 to 2533). The BamHI to BglI fragment is derived from pBR328 (375 to 2422) with an additional deletion between the SalI and the AvaI sites (651 to 1425) following the addition of a SalI linker to the AvaI site. The sequence from the BglI site originates from the β-lactamase gene of pSP64.

Example 2: Mutant pUK-GK (deletion of amino acids 11 to 135)

Mutant pUK-GK (deletion of amino acids 11 to 135) was constructed by first removing the region between the BssHII site and the BalI site (amino acids 3 to 149) of pEE7.pUK and then inserting appropriate oligonucleotides to construct the mutant gene. The oligonucleotides shown below were annealed together and the resulting double-stranded DNA fragment of 115 base pairs was isolated from an agarose gel and ligated to the BssHII-BalI fragment of pEE7.pUK, to form pEE7.pUK-GK.

Sense-strand Oligonucleotides (5' to 3')

    1. CGCGCCTGCTTCTCTGCGTCCTGGTCGTGAGCGAC
    2. TCCAAAGGCAGCAATGAACTTCATCAAGTTCCAT
    3. CGAACAAGCCCTCCTCTCCTCCAGAAGAATTAAAATTTCAGTGTGG

Antisense-strand Oligonucleotides (5' to 3')

    4. CCACACTGAAATTTTAATTCTTCTGGAGGAGAGGA
    5. GGGCTTGTTCGATGGAACTTGATGAAGTTCATT
    6. GCTGCCTTTGGAGTCGCTCACGACCAGGACGCAGAGAAGCAGG

Example 3: Synthesis of mutants PUK-CAR, PUK-DECAR, and PUK-CS

| Effect of mutation | Mutant Label |
|---|---|
| 2. $His_{56}$ Ser, $Ala_{77}$ Ser | PUK-CAR |
| 3. $Asn_{302}$ Gln | PUK-DECAR |
| 4. $Ile_{160}$ Lys | PUK-CS |

For the other modified u-PA proteins described above, mutagenesis was achieved employing the procedure described by Kramer et al. ((1984) Cell 38: 879-887). The following table indicates the oligomers employed for mutagenesis and sequencing. Mutagenesis primers 5' to 3'

$His_{56}$ to Ser: [216]GGGGAATGGTAGCTTTTACCGAG[238]

$Ala_{77}$ to Ser: [278]CTGGAACTCTTCCACTGTCCTTC[301]

$Asn_{302}$ to Gln: [951]CTTTGGAA AAGAGCAGTCTACCGACTATC[979] $Ile_{160}$ to Lys: [528]CTTTAAGATTAAGGGGGGAGAATTC[552]

Mutagenesis of M13/u-PA recombinants was performed using purified templates by annealing and extending the appropriate primer with Klenow enzyme or T4 DNA polymerase. Following transfection of HB2154/2151 cells (Zoller and Smith, supra), plaques were grown at a density of 200-500/plate and were lifted onto filters and screened by hybridization with the appropriate mutagenesis primer or probe. For each mutant, between 20 and 60 percent of these plaques were initially identified as putative positive clones. The DNA sequence of sereval such clones was determined using suitable primers and template preparations.

Example 4: Mammalian Cell Transfection

COS-1 cells (Gluzman, Cell (1981) 23: 174) were transfected with the u-PA expression plasmids using a modification of the procedure described by Lopata et al. (Nucleic Acids. Res. (1984) 12: 5707-5717).

The following solutions were used: Dulbecco's modified Eagle Medium (DMEM); 0.25 M Tris-HCl (pH 7.5); TBS (0.025 M Tris-Cl pH 7.4, 0.1375 M NaCl, 5 mM KCl, 0.7 mM $CaCl_2$, 0.5 mM $MgCl_2$, 0,6 mM $Na_2HPO_4$); HBS (137 mM NaCl, 5 mM KCl 0,7 mM $Na_2PO_4$, 9 mM glucose, 21 mM Hepes-NaOH, pH 7.1); Img/ml DEAE-dextran in TBS; and DEAE-Tris, made from 20 ml DMEM and 5 ml of 0.25 M Tris pH 7.5.

Approximately $6 \times 10^6$ cells per 150 mm Petri dish were washed twice in DMEM and transfected with 35 $\mu$g super-coiled plasmid DNA by incubation for 6-8 hours at 37°C in 6.5 ml of Dextran solution. Dextran solution is a 1:4 mixture of 1 mg/ml DEAE-Dextran in TBS and DMEM-Tris.

The DNA-containing solution was then removed and the cells were subjected to a DMSO shock by incubation at 37°C for 2 mins in 10% DMSO in HBS. Cells were then washed in DMEM and cultured for 2-3 days in DMEM + 10% foetal calf serum.

Cells were then either washed in serum-free medium and incubated in 25 ml fresh serum-free medium for approximately 21 hours or were kept in serum-containing medium for a further 21 hours. Spent medium from more than one Petri-dish was pooled for each treatment and assayed for u-PA.

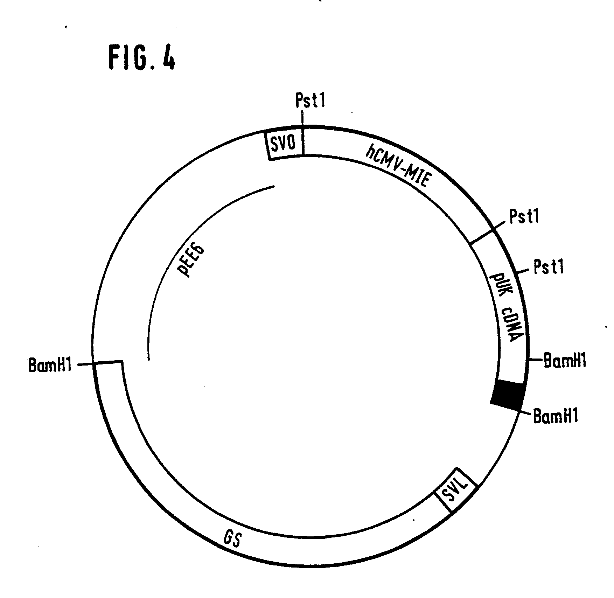

In order to generate permanent mutant u-PA protein expressing cell lines the HCMV-IE promoter enhancer (the Pst-1 M fragment of HCMV) was first inserted at the Pst-1 site upstream of the u-PA open reading frame in pEE7pUK of form pEE7HCMVpUK. A glutamine-synthetase (GS) gene was also introduced into each u-PA expression plasmid to act as a dominant selectable marker in mammalian cultured cells. Specifically a 5.5 kb Pvul-BamHI fragment of pSVLGS.1 (Bebbington and Hentschel in DNA Cloning Vol. III Chapter 8 ed. D. Glover (1987)) containing the GS gene was inserted into the BamHI site immediately down-stream of the SV40 Early polyadenylation signal in pEE7HCMVpUK and each mutant derivative plasmid, after addition of a BamHI synthetic oligonucleotide linker to the Pvul site, using standard techniques. The representative expression plasmid pEE7HCMVpUK.GS containing the wild-type u-PA coding sequences is shown in Fig. 5.

GS-containing plasmids were introduced into CHO-KI cells by a calcium phosphate-mediated tranfection procedure essentially as described by Gorman (DNA Cloning Vol.II, (Chapter 6) ed. D. Glover, (1985)).

Solutions used are 2 M $CaCl_2$, 2 x HBS (containing 290 mM NaCl, 50 mM Hepes, 2.8 mM $Na_2HPO4$) and 15% glycerol in HBS.

$1-2 \times 10^6$ cells were seeded per 90 mm Petri dish the day before transfection in the non-selective culture medium GMEM-S described by Bebbington and Hentschel (supra). On the day of transfection, the following were mixed in a sterile plastic tube (volumes are per dish transfected) : 62 $\mu$l $CaCl_2$, 10 $\mu$g DNA and distilled water to 0.5 ml.

This mixture was slowly added to 0.5 ml 2 x HBS in a second tube to form a $CaPO_4$-DNA co-precipitate

which was then mixed vigorously by vortexing.

Cells were washed once with serum-free GMEM-S medium and the CaPO$_4$-DNA co-precipitate added to the cells together with 1 ml of serum-free medium and incubated for 3.5 - 4 hours at 37°C. After removal of the DNA, the cells were subjected to a glycerol shock by addition of 3 ml of 15% glycerol in HBS for 1.5 minutes, washed in serum-free medium and allowed to recover for 24 hours in serum-containing GMEM-S medium. Selection for expression of the introduced GS gene was then applied by addition of 25 µM methionine sulphoximine (MSX; Sigma). MSX-resistant colonies were isolated after 2-3 weeks, transferred to 24-well dishes and assayed for u-PA cross-reactive material by ELISA.

Cell lines secreting high levels of plasminogen activator were grown in mass culture and the secretion rates of the highest producing cell lines for each mutant protein, estimated by ELISA assay, (or by amidolytic assay for pUK-GK) are shown in Table 1.

Table 1

| Mutant Protein | Secretion Rate (pg/cell/day) |
|---|---|
| PUK | 5 |
| PUK-DECAR | 6 |
| PUK-CAR | 10 |
| PUK-CS | 9,5 |
| PUK-GK | 11 |

Example 5: Preparation of mutant protein with plasminogen activator activity

Prior analysis: All serum free COS supernatants were made 0,01% Tween 80 TM and 0,01% NaN$_3$ and frozen in 1 ml portions at -25°C. The single chain form of the molecules was assessed by SDS polyacrylamide gel electrophoresis.

Example 6: Comparison of mutants (1) to (4) with wild-type u-PA

Clot lysis test (CLT)

The clot lysis test was performed using a "KC 10-Koagulometer " (Amelung, FRG) which has been modified in order to record the lysis time instead of the coagulation time.

All reagents were preincubated at 37°C. The u-PA standard (WHO 66/46), the polypeptide test samples and fibrinogen were diluted to the required concentrations in a Tris-buffer (0.50 M) containing 1% Haemaccel TM (pH 7.4). 0.2 ml of the u-PA standard or of the samples were mixed with 0.2 ml human plasminogen (10 CTA/ml), 0.5 ml bovine fibrinogen (0.20%) and finally 0.1 ml thrombin (10 IU/ml). The lysis time was recorded and expressed in u-PA units using a calibration curve. The activity of each mutant protein was analyzed at least three times, and the average was calculated. Four dilutions of the u-PA standard were run with each of the samples to be analyzed. The same assay was performed with the mutant single chain proteins after conversion in the two chain form by incubation for 2 min. with 1 CTA plasmin/ml. The reaction was stopped by addition of 50 mg aprotinin-sepharose/ml. The mixture was incubated for 15 min and after completion of the reaction, the aprotinin-sepharose was discarded and the activated samples assayed in the CLT. (Table 2)

Table 2

| | CLT (1) prior plasmin treatment | CLT (2) after plasmin treatment | Ratio 2/1 |
|---|---|---|---|
| | IU/ml ± sigma(%) | IU/ml ± sigma(%) | |
| PUK-GK | 51,6 ± 8,4 % | 215,0 ± 11,3 % | 4,2 |
| PUK-CAR | 15,0 ± 12,1 % | 60,2 ± 1,4 % | 4,0 |
| PUK-DECAR | 19,1 ± 7,4 % | 19,2 ± 8,1 % | 1,0 |
| PUK-CS | 12,8 ± 14,3 % | 21,0 ± 2,7 % | 1,6 |
| UK | 985 ± 13,2 % | 950 ± 15,9 % | 0,97 |
| WHO-Std | | | |

Amidolytic assay

All reagents were preincubated at 37° C. Where indicated, the mutant single chain proteins (0.7 ml) were converted in the two chain form by incubation for 2 min. with plasmin (0.1 ml of a plasmin solution containing 10 CTA/ml) at 37° C. The reaction was stopped by addition of 0.1 ml of a aprotinin solution containing 500 KIU/ml. The generated urokinase activity was measured after addition of the chromogenic substrate S-2444 (0.1 ml of a 3 mM water solution) at 405 nm. The activity of each mutant protein prior and after plasmin treatment was analyzed at least three times and compared with the activity of the urokinase standard. (Table 3).

Table 3

| | I | II | Ratio II/I |
|---|---|---|---|
| | IU/ml ± sigma(%) prior plasmin treatment | IU/ml ± sigma(%) after plasmin treatment | |
| PUK-GK | 7,0 ± 10,0% | 291 ± 3,1% | 41,6 |
| PUK-CAR | 3,3 ± 13,2% | 61,7 ± 3,7% | 18,7 |
| PUK-DECAR | 4,1 ± 7,0% | 33,3 ± 6,5% | 8,1 |
| PUK-CS | 2,5 ± 14,2% | 11,9 ± 9,3% | 4,8 |
| UK | 1273 ± 2,6 % | 1280 ± 2,6 % | 1,0 |
| WHO-Std | | | |

Inhibiton test

The susceptibility of the mutant protein to be inhibited by the plasminogen activator inhibitor type 2 (PAI-2) was analyzed as followed: 0.8 ml of the activated mutant proteins (see amidolytic assay) containing 6.25 units, as determined in the amidolytic assay, were mixed with a solution (0.2 ml) containing S-2444 and PAI-2. The inhibition susceptibility of each mutant protein was tested at final substrate concentrations of 0.1; 0.2 and 0.3 mM and at final PAI-2 concentrations of 10, 20, 40, 80 and 160 urokinase inhibitory units. The inhibitory constant ($K_1$) has been calculated for each mutant assuming that 1 mg PAI-2 correspond to 100 000 urokinase inhibitory units. The $K_i$ obtained for each mutant represents the average of at least three independent measurements:

| Mutant | $K_I$ | |
|--------|-------|---|
| PUK-GK | 2.12 | $10^{-9}$ |
| PUK-CAR | 1.06 | $10^{-9}$ |
| PUK-DECAR | 3.71 | $10^{-9}$ |
| PUK-CS | 18.9 | $10^{-9}$ |
| Wild Type | 1.31 | $10^{-9}$ |

Thrombin inactivation assay

0.1 ml of the mutant single chain proteins containing 10 IU/ml according to the amidolytic assay were mixed with a thrombin solution containing 0.5 IU/ml and incubated for 0, 30, 70, 120, 200 and 300 min. at 37° C. After addition of a buffer solution (0.5 ml) containing 0.1 M Tris, 0.1% Tween 80$^{(R)}$ pH 7.5 the single chain proteins were converted to the two chain form by addition of 0.1 ml plasmin (10 CTA/ml, incubation time 2 min. at 37° C). Before testing the plasmin activity was neutralized by addition of 0.1 ml aprotinin (500 KIU/ml) and incubation for 2 min. at 37° C. Finally 0.1 ml S-2444 (3 mM) was added and the residual urokinase activity was determined.

| Mutant | $t_{50\%}$ inactivation |
|--------|------------------------|
| PUK-GK | 65 min. |
| PUK-CAR | 20 min. |
| PUK-DECAR | 22 min. |
| PUK-CS | 65 min. |
| Wild Type | 20 min. |

Legend

Figure 1:

Restriction Map of pEE7 pUK
All sites recognized by enzymes which cut once or twice are shown.
pA - SV40 polyadenylation signal
SVORI - SV40 origin of replication

Figure 2:

Restriction Map of a Partial pUK cDNA in lambdagt 10 Cloning vector (Clone H1.2)
Arrows indicate the DNA fragments sequenced in M13 or in plasmid vectors and the direction of the sequencing.
Numbers indicate fragment sizes in base-pairs.

Figure 3:

Restriction map of pEE6
pEE6 is the basic plasmid used to assemble u-PA expression vectors.

Figure 4:

Restriction map of pEE7 hCMV pUKGS, used for expression of u-PA and mutant protein in permanent transfected CHO-K1 cell lines.

Pst-1 and BamH1 sites are shown to provide reference points.

**Claims**

1. A mutant of human urinary plasminogen activator having at least one, or a combination of, or all of the following mutations

(i) change of the naturally occurring glycosylation site to prevent glycosylation, preferably $Asn_{302}$ to Gln

(ii) deletion of part or total of amino acids 1 through 135, preferably deletion of amino acids 11 through 135

(iii) conservative or non-conservative substitution of the $Ile_{160}$, preferably by Lys or Arg

(iiii) substitution of at least one amino acid to create new glycosylation site(s), preferably the substitution of $His_{56}$ to Ser and/or $Ala_{77}$ to Ser.

2. A mutant according to claim 1, having at least on amino acid substitution at the naturally occurring glycosylation site represented by Asn-Ser-Thr so as to prevent glycosylation.

3. A mutant according to claim 1 or 2, wherein $Asn_{302}$ is substituted so as to prevent glycosylation.

4. A mutant according to claim 1, 2 or 3, wherein $Asn_{302}$ is substituted by Gln so as to prevent glycosylation.

5. A mutant according to claim 1, wherein $Ile_{160}$ is substituted by Arg or Lys.

6. A mutant according to claim 1, wherein $Thr_{304}$ is replaced by a non-conservative amino acid.

7. A mutant according to claim 6, wherein $Thr_{304}$ is replaced by Gly, Ala or Val.

8. A mutant according to claim 1, 2, 3, 4, 5, 6 or 7 having one or more substitution at the cleavage site in the region from amino acids 155 to 162 or in the region from 159 to 162.

9. A mutant according to claim 8 wherein the $Ile_{160}$ is substituted, preferably by Arg or Lys.

10. A mutant according to anyone of the preceeding claims, wherein part or all of the sequence $Lys_{11}$ to $Lys_{135}$ is lacking.

11. A mutant according to anyone of the preceeding claims as a pharmaceutical.

12. A DNA sequence encoding anyone of the mutants of claims 1 to 10.

13. An expression construct or vector containing a DNA sequence according to claim 12.

14. A pro- or eucaryotic cell transformed with an expression construct or a vector according to claim 13.

15. A method to produce a mutant of human urinary plasminogen activator by transforming a suitable pro-or eukaryotic host with an expression construct according to claim 13, inducing synthesis of the mutant and purifying it following the Contracting States: ES, GR

1. A method to produce a mutant of human urinary plasmidnogen activator by transforming a suitable pro- or eukaryotic host with an expression construct containing the cDNA sequence of said mutant, inducing expression of said cDNA sequence and purifying the expression product from the cellular proteins, said mutant being characterized by at least one, or a combination of, or all of the following mutations

(i) change of the naturally occurring glycosylation site to prevent glycosylation, preferably $Asn_{302}$ to Gln

(ii) deletion of part or total of amino acids 1 through 135, preferably deletion of amino acids 11 through 135

(iii) conservative or non-conservative substitution of the $Ile_{160}$, preferably by Lys or Arg

(iiii) substitution of at least one amino acid to create new glycosylation site(s), preferably the substitution of $His_{56}$ to Ser and/or $Ala_{77}$ to Ser.

2. A method according to claim 1, said mutant being characterized by having at least one amino acid substitution at the naturally occuring glycosylation site represented by Asn-Ser-Thr so as to prevent glycosylation.

3. A method according to claim 1 or 2, said mutant being characterized by substitution of $Asn_{302}$ so as to prevent glycosylation.

4. A method according to claim 1, 2 or 3, said mutant being characterized by substitution of $Asn_{302}$ by Glu so as to prevent glycosylation.

5. A method according to claim 1, said mutant being characterized by substitution of $Ile_{160}$ by Arg or Lys.

6. A method according to claim 1, said mutant being characterized by substitution of $Thr_{304}$ by a non-

conservative amino acid.

7. A method according to claim 6, said mutant being characterized by substitution of $Thr_{304}$ by Gly or Ala or Val.

8. A method according to one of claims 1 through 7, said mutant being characterized by having one or more substitution at the cleavage site in the region from amino acids 155 to 162 or in the region from 159 to 162.

9. A method according to claim 8, said mutant being characterized by substitution of $Ile_{160}$ by preferably Arg or Lys.

10. A mutant according to one of claims 1 through 9, said mutant being characterized by deletion of part or all of the sequences $Lys_{11}$ to $Lys_{135}$.

# FIG. 1

BcII<SacI
HpaI<BsmI
BsmI
BamHI
NaeI
Eco47III
AvaI<SalI
ApaLI
HgiEII
GsuI<Eco31I
PvuI
ScaI
Asp700
ApaLI
SspI
AatII
BbvII
AvrII<StuI
SfiI
NcoI
NsiI
NsiI
HindIII<PstI
BssHII
AflIII
ScaI
NcoI<NaeI
BglII
PstI
GsuI
BalI
HgiEII
AflIII
BclI
Eco47III
BspMII
PpuMI
BamHI
PpuMI

pA

PUK

SVORI

pEE 7 PUK

4433

FIG. 2

pUK CODING SEQUENCE

# FIG. 3

# FIG. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 288 435  (CIBA-GEIGY AG)<br>* Claims 2,3,10,11,15-17 *<br>--- | 1-4,8,<br>11-15 | C 12 N   15/00<br>C 12 N    9/72 |
| X | EP-A-0 266 032  (BEECHAM GROUP PLC)<br>* Claims *<br>--- | 1,10-15 |  |
| X | THROMBOSIS AND HAEMOSTASIS, vol. 58, no. 1, 1987, page 216, abstract no. 803, Stuttgart, DE; W.A. GÜNZLER et al.: "Characterization of recombinant human single-chain low molecular weight urokinase (RE-SC-LUK)"<br>* Abstract *<br>--- | 1,11-15 |  |
| X | EUROPEAN JOURNAL OF BIOCHEMISTRY, vol. 177, no. 3, 1988, pages 575-582, Berlin, DE; H.R. LIJNEN et al.: "Structural and functional characterization of mutants of recombinant single-chain urokinase-type plasminogen activator obtained by site-specific mutagenesis of Lys158, Ile159 and Ile160"<br>* Abstract *<br>----- | 1,5,8,9<br>,11-15 | TECHNICAL FIELDS SEARCHED (Int. Cl.5)<br><br>C 12 N |

The present search report has been drawn up for all claims

| Place of search | Date of completion of the search | Examiner |
|---|---|---|
| THE HAGUE | 30-06-1989 | HUBER-MACK A. |

EPO FORM 1503 03.82 (P0401)